(19) Europäisches Patentamt
European Patent Office
Office européen des brevets

(11) Veröffentlichungsnummer : **0 044 440**
**B1**

(12) # EUROPÄISCHE PATENTSCHRIFT

(45) Veröffentlichungstag der Patentschrift : **18.01.84**

(21) Anmeldenummer : **81105003.8**

(22) Anmeldetag : **27.06.81**

(51) Int. Cl.³ : **C 07 C 47/42**, C 07 C119/06, C 07 C 45/42

(54) **Verfahren zur Herstellung von alpha- und beta-Cyclocitral sowie die N-Methylaldimine dieser Verbindungen.**

(30) Priorität : **22.07.80 DE 3027689**

(43) Veröffentlichungstag der Anmeldung : **27.01.82 Patentblatt 82/04**

(45) Bekanntmachung des Hinweises auf die Patenterteilung : **18.01.84 Patentblatt 84/03**

(84) Benannte Vertragsstaaten : **CH DE FR GB LI NL**

(56) Entgegenhaltungen :
**DE-C- 123 747**
**US-A- 3 391 192**
**Chemical Abstracts Band 75, Nr. 5, 2 August 1971 Columbus, Ohio, USA R.N. GEDYE et al. "Preparation of beta-cyclocitral" Seite 444, Spalte 1, Abstract Nr. 35206x**
**Chemical Abstracts Band 56, Nr. 1, 8 Januar 1962 Columbus, Ohio, USA V.B. MOCHALIN et al. "Synthesis of aldehydes, ketones and alcohols of the isoprene series" Spalten 509f to 510c**
**CHEMISCHES ZENTRALBLATT Band 99, Nr. 6, 1928 II, Berlin A. SKITA et al. "Basenbildung aus Carbonylverbindungen" Seiten 647 bis 649**
**Ber.Dtsch.Chem.Ges. 61 (1928), S. 1452**

(73) Patentinhaber : **BASF Aktiengesellschaft**
**Carl-Bosch-Strasse 38**
**D-6700 Ludwigshafen (DE)**

(72) Erfinder : **Janitschke, Lothar, Dr.**
**Berner Weg 34**
**D-6700 Ludwigshafen (DE)**
Erfinder : **Hoffmann, Werner, Dr.**
**Ringstrasse 11C**
**D-6701 Neuhofen (DE)**

**EP 0 044 440 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von α- und β-Cyclocitral sowie die N-Methylaldimine dieser Verbindungen

Die vorliegende Erfindung betrifft ein verbessertes Verfahren zur Herstellung von Cyclocitralen der allgemeinen Formel I

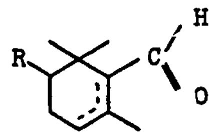

(I)

in welcher R für Wasserstoff oder die Methylgruppe steht und in welcher die gestrichelt eingezeichneten Bindungen eine Doppelbindung in 1- oder 2-Stellung des Cyclohexenringes bedeuten.

Ferner betrifft die Erfindung die bei diesem Verfahren als Zwischenprodukt auftretenden N-Methylaldimine dieser Cyclocitrale mit der allgemeinen Formel II

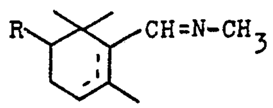

(II)

Aus der DE-PS 123 747 ist ein Verfahren zur Herstellung von Cyclocitral bekannt, gemäß dem Citral mit Anilin oder Ethylamin kondensiert und das erhaltene Kondensationsprodukt mit konzentrierter Säure cyclisiert wird.

Nach den Untersuchungen in einer jüngeren Arbeit (Helv. Chim. Acta, Band 28 (1951) S. 265-273) erhält man das Cyclocitral nach dem Verfahren dieser Patentschrift jedoch nur in Ausbeuten von wenigen Prozent. Als Verbesserung wird in dieser Arbeit vorgeschlagen, das Cyclocitral über das Cycloanil herzustellen und bestimmte Reaktionsbedingungen zu beachten, nämlich das Anil zur Cyclisierung mit Ether zu verdünnen, und diese Reaktion bei −15 °C mit Hilfe einer etwas Wasser enthaltenden Schwefelsäure vorzunehmen. Trotzdem erhielt man hierbei nur Cyclocitralausbeuten von 60 bis 70 %.

Auch gemäß einem Verfahren in Can. J. Chem. *49* (1971). S. 1764 bis 1766, erhält man β-Cyclocitral indem man Citralanil mit Schwefelsäure cyclisiert. Die Ausbeuten betragen 50 bis 60 %, bezogen auf eingesetztes Citral.

Die nach den bekannten Verfahren erzielbaren Ausbeuten sind jedoch technisch immer noch unbefriedigend, weshalb es Aufgabe der Erfindung war, die für zahlreiche organische Synthesen wichtigen Cyclocitrale I auf wirtschaftlichere Weise und in höheren Ausbeuten als bisher zugänglich zu machen.

Es wurde nun überraschenderweise gefunden, daß man die Cyclocitrale der allgemeinen Formel I

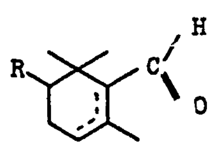

(I)

in welcher R für Wasserstoff oder die Methylgruppe steht und in welcher die gestrichelt gezeichneten Bindungen eine Doppelbindung in 1- oder 2-Stellung des Cyclohexenrings bedeuten, sehr vorteilhaft erhält, wenn man die offenkettigen Aldehyde der allgemeinen Formel III

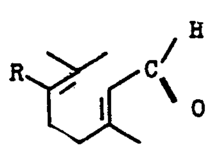

(III)

bei −20 bis 60 °C, vorzugsweise 0 bis 40 °C, mit Methylamin zu deren N-Methylaldiminen umsetzt, diese bei −30 bis 30 °C, vorzugsweise −20 bis 0 °C, mit einem mehrfach molaren Überschuß von konzentrierter Schwefelsäure zu den Cyclocitral-N-methylaldiminen der allgemeinen Formel II

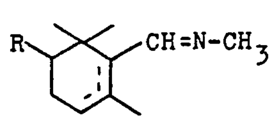

(II)

cyclisiert und die letztgenannten Verbindungen nach Abtrennung aus ihren Reaktionsgemischen oder

ohne sie zuvor zu isolieren in an sich bekannter Weise zu den Cyclocitralen I hydrolysiert.

Gemäß dem erfindungsgemäßen Verfahren erhält man das Cyclocitral (2,6,6-Trimethyl-cyclohex-1 bzw. -2-en-1-carboxaldehyd) in Ausbeuten von 80 bis 90 %, bezogen auf eingesetztes Citral. Diese außerordentlich guten Ergebnisse waren überraschend, da die Citral-N-methyl-aldimine bekanntermaßen zersetzlich sind (vgl. Ber. Dtsch. Chem. Ges. *61* (1928), S. 1452) und man wohl deshalb bisher immer den Weg über das wesentlich teurere Citralanil gewählt hat.

Als Ausgangsmaterialien eignen sich die Verbindungen III sowohl in ihrer cis-Form als auch in der trans-Form, so daß man z. B. die cis-trans-Isomerengemische von Citral oder die Gemische der entsprechenden 6-Methyl-homologen (im folgenden als Methylcitral bezeichnet) einsetzen kann.

Zur Herstellung der N-Methyl-aldimine setzt man je ein Mol III zweckmäßigerweise bei Raumtemperatur oder leicht erhöhter Temperatur, also etwa zwischen 20 und 50 °C mit 2 mol Methylamin um. Danach trennt man die sich hierbei bildende wäßrige Phase ab und entfernt das überschüssige Methylamin destillativ bei etwa 20 bis 50 °C bei einem Druck von 10 bis 20 mbar.

Die rohen N-Methyl-aldimine, die bei dieser Arbeitsweise in praktisch quantitativer Ausbeute anfallen, werden sodann der Ringschlußreaktion unterworfen, indem man sie bei − 20 bis 0 °C in konzentrierte Schwefelsäure eintropft. Die Schwefelsäure kann bis zu etwa 10 Gew.-% Wasser enthalten. Die besten Ergebnisse erzielt man jedoch mit konzentrierter Schwefelsäure. Das Molverhältnis von Aldimin zur Schwefelsäure (auf 100 % berechnet) beträgt im allgemeinen 1 : 2 bis 1 : 10, vorzugsweise 1 : 4 bis 1 : 7.

Sowohl bei der Herstellung der Aldimine von III als auch bei deren Cyclisierung zu den Verbindungen der Formel II können Lösungsmittel wie Diethylether und Hexan mitverwendet werden. Jedoch ist es ein Vorteil des erfindungsgemäßen Verfahrens, daß man ohne Lösungsmittel nahezu gleiche Ausbeuten erzielt.

Zur Isolierung der cyclischen Verbindungen der Formel II neutralisiert man die bei der Ringschlußreaktion anfallenden schwefelsauren Lösungen bei relativ niedrigen Temperaturen — etwa − 20 bis 40 °C — um die Hydrolyse der Schiffschen Basen zu den Aldehyden zu unterbinden. Es empfiehlt sich, die schwefelsauren Lösungen zunächst mit etwa der gleichen Menge Eis zu versetzen und die so erhaltenen wäßrigen sauren Lösungen sodann zu neutralisieren. Hierzu verwendet man möglichst billige Basen wie Natronlauge. Aus den neutralen oder schwach alkalischen wäßrigen Lösungen kann man die Cyclocitral-α-methylaldimine wie üblich isolieren, z. B. durch Wasserdampfdestillation, durch Extraktion mit einem Lösungsmittel wie Diethylether, Methyl-tert.-butylether, n-Hexan oder Toluol oder mit Hilfe einer Kombination beider Methoden.

Man erhält die Cyclocitral-N-methyl-aldimine der Formel II gemäß gaschromatographischer Analyse des Reaktionsgemisches in Ausbeuten von 65 bis 75 %, bezogen auf das eingesetzte Citral, in Form von Gemischen aus den 1-en- und den 2-en-Isomeren, also als Derivate des α- und β-Cyclocitrals bzw. deren definitionsgemäßen Methylhomologen.

Zur Herstellung der Cyclocitrale der Formel I ist es nicht erforderlich, die Aldimine aus ihren schwefelsauren Lösungen zu isolieren, vielmehr ist es zweckmäßig, die Neutralisation bei höheren Temperaturen — etwa 20-80 °C, vorzugsweise 30 bis 50 °C — nur soweit vorzunehmen, bis die Lösung noch schwach sauer ist und einen pH-Wert von 2 bis 6, vorzugsweise 3 bis 6 hat. Unter diesen Bedingungen, ggf. nach einiger weiterer Zeit der Erwärmung, hydrolysieren die Schiffschen Basen vollständig zu den freien Aldehyden.

Die Aufarbeitung auf die Cyclocitrale erfolgt wie üblich durch Wasserdampfdestillation oder durch Extraktion mit Diethylether, Methyl-t-butylether oder n-Hexan.

Man erhält Isomerengemische von 2-en und 1-en-Verbindungen (α- und β-Cyclocitral), deren Trennung durch fraktionierte Destillation bei 0,1 bis 20 mbar relativ einfach möglich ist.

Das Verhältnis von den 1-en- zu den 2-en-Cyclocitralen läßt sich durch die Aufarbeitungsbedingungen beeinflussen. Bei − 20 bis 40 °C, insbesondere bei 0 bis 30 °C und in schwach saurer Lösung (pH 3,5) erhält man ein Verhältnis von 1-en- zu 2-en-Verbindungen von etwa 40 : 60 bis 20 : 80.

Arbeitet man ohne Neutralisation auf, indem man das Cyclisierungsgemisch auf Eis gibt und die stark saure Mischung mit Wasserdampf destilliert, beträgt das Verhältnis 80 : 20 bis 85 : 15, jedoch können sich hierbei merkliche Mengen (etwa 10-15 %) der Umlagerungsprodukte 1-Acetyl-4,4-dimethyl- bzw. 1-Acetyl-4,4,5-trimethyl-cyclohex-1-en bilden. Die Herstellung der Cyclocitrale nach dem erfindungsgemäßen Verfahren kann kontinuierlich oder diskontinuierlich erfolgen.

Die Cyclocitrale der Formel I sind wichtige Zwischenprodukte für die Herstellung von Duftstoffen und Carotinoiden wie Vitamin A und damit verwandten Produkten. Das gleiche gilt für die N-Methylaldimine der Formel II, welche in ihrem reaktiven Verhalten dem der freien Aldehyde gleichen und deshalb häufig unmittelbar, d. h. ohne zuvor isoliert werden zu müssen, weiter umgesetzt werden können. Weitere wichtige Synthesemöglichkeiten liegen in der Hydrierung der Verbindungen der Formeln I und II, die zu den entsprechenden Alkoholen bzw. Aminen führt. Mit Hilfe des erfindungsgemäßen Verfahrens ist es möglich, Cyclocitral, dessen Methylhomologes bzw. die Schiffschen Basen dieser Verbindungen in guten Ausbeuten und hoher Reinheit herzustellen.

## Beispiel 1

a) Herstellung von Citral-N-methylaldimin

In 310 g (rund 2 mol) Citral, welches als handelsübliches cis-trans-Isomerengemisch vorlag, wurde bei 20 bis 25 °C innerhalb von 45 Minuten 94 g (rund 3 mol) Methylamin eingeleitet, wobei sich eine wäßrige Phase abschied. Nach weiteren 10 Minuten wurde die Wasserphase weitgehend (rund 37 g) abgetrennt und der organische Rückstand bei 20 °C und 20 mbar teilweise vom überschüssigen Methylamin befreit. Die Ausbeute an Citral-N-methyl-aldimin war praktisch quantitativ.

b) Cyclisierung des Citral-N-methyl-aldimins

Das gemäß Beispiel 1a hergestellte rohe Citral-N-methyl-aldimin wurde im Verlauf von einer Stunde unter Stickstoffatmosphäre und unter starkem Rühren bei − 10 bis − 15 °C in 1 000 g konzentrierter Schwefelsäure eingetropft, wonach das Gemisch noch 20 Minuten bei − 15 °C gerührt wurde.

c) Herstellung von Cyclocitral

Das gemäß Beispiel 1b erhaltene schwefelsaure Reaktionsgemisch wurde zunächst mit 1,2 kg Eis und danach bei 40 °C mit soviel 50 gew.-%iger Natronlauge versetzt, bis ein pH-Wert von etwa 3,5 erreicht wurde. Im Laufe einer Stunde stieg der pH-Wert, ohne daß weitere Natronlauge zugegeben wurde auf 6,8 an. Das Gemisch wurde sodann auf pH 7 eingestellt, wonach die wäßrige Phase und auskristallisiertes Natriumsulfat abgetrennt wurden. Die organische Phase wurde anschließend der Wasserdampfdestillation unterworfen und aus dem Kondensat (rund 5 Liter) wurde das Cyclocitral durch dreimalige Extraktion mit je 250 ml Diethylether extrahiert. Nach Trocknung der etherischen Lösung und Entfernung des Ethers blieben 270,1 g Cyclocitralgemisch zurück. Dies entspricht einer Ausbeute von 86 %, bezogen auf eingesetztes Citral.

Nach gaschromatographischer Analyse bestand dieses Cyclocitralgemisch aus 62 Gew.-% α-Cyclocitral, 36 Gew.-% β-Cyclocitral und 2 % nicht näher bestimmten Verbindungen. Durch fraktionierte Destillation wurde das Isomerengemisch in reines α-Cyclocitral (Kp 39 °C bei 0,4 mbar) und reines β-Cyclocitral (Kp 51-52 °C bei 0,2 mbar) aufgetrennt.

## Beispiel 2

Herstellung von α-Cyclocitral-N-methyl-aldimin (2,6,6-Trimethylcyclohex-2-en-1-carboxaldehyd-N-methylaldimin)

Ein wie in Beispiel 1a beschrieben hergestelltes rohes Citral-N-methylaldimin (347 g) wurde in 300 ml n-Hexan gelöst und unter den Bedingungen gemäß Beispiel 1b mit 1,2 kg konz. Schwefelsäure cyclisiert. Anschließend wurde das schwefelsaure Gemisch auf 2 kg Eis gegeben und bei 0 bis 10 °C (Außenkühlung mit Trockeneis/Aceton) mit 50 gew.-%iger Kalilauge neutralisiert. Die organische Phase 3x mit je 100 ml n-Hexan extrahiert. Nach üblicher Aufarbeitung der vereinigten organischen Phasen fiel das Cyclocitral-N-methyl-aldimin bei der fraktionierten Destillation in 59 %iger Ausbeute an, bezogen auf eingesetztes Citral. Kp = 38 °C bei 0,3 mbar.

Nach gaschromatographischer Analyse setzt sich dieses Produkt aus 85 Gew.-% α-Cyclocitral-N-methylaldimin und 13 Gew.-% α-Cyclocitral zusammen.

## Beispiel 3

Herstellung von vorwiegend α-Cyclocitral (2,6,6-Trimethylcyclohex-2-en-1-carboxaldehyd)

282 g des α-Cyclocitral-N-methyl-aldimins wurden bei 10 bis 25 °C mit 50 gew.-%iger wäßriger Schwefelsäure versetzt bis das Gemisch einen pH-Wert von 3,5 aufwies. Nach 12-stündigem Rühren bei Raumtemperatur wurde das Gemisch mit 10 gew.-%iger Natronlauge neutralisiert, der Wasserdampfdestillation unterworfen und gemäß Beispiel 1c aufgearbeitet. Man erhielt 200,3 g Cyclocitral, entsprechend einer Ausbeute von 81,2 %. Nach gaschromatographischer Analyse erhielt dieses Gemisch 79 % an dem α-Cyclocitral und 20 % β-Cyclocitral.

## Beispiel 4

Herstellung von Methyl-α-cyclocitral-N-methyl-aldimin (2,5,6,6-Tetramethyl-cyclohex-2-en-1-carboaldehyd-N-methylaldimin)

In 200 g (1 mol) 3,6,7-Trimethyl-octa-2,6-dien-1-al (mindestens 83 %ig) wurden im Laufe von 60 Minuten bei 20 bis 25 °C 47 g (1,5 mol) Methylamin eingeleitet, wonach das Gemisch noch 30 Minuten bei Raumtemperatur gerührt wurde. Die wäßrige Phase wurde abgetrennt und die organische am Rotationsverdampfer (20 °C bei 20 mbar) vom überschüssigen Methylamin befreit. Zurück blieben 195 g des rohen Aldimins. Das rohe Aldimin wurde analog Beispiel 1b mit 450 g konzentrierter Schwefelsäure versetzt und danach auf 3 kg Eis gegossen. Nach Zusatz von 1 Liter n-Hexan wurde diese wäßrig-organische

Mischung bei 0 bis 5 °C mit 50 gew.-%iger Natronlauge neutralisiert, wonach das Aldimin analog Beispiel 2 isoliert wurde. Man erhielt 108 g entsprechend einer Ausbeute von 60 %. (Kp = 50 °C bei 0,6 mbar ; Kp = 96 °C bei 17 mbar).

Beispiel 5

Herstellung von überwiegend 5-Methyl-α-cyclocitral (2,5,6,6-Tetramethyl-cyclohex-2-en-1-carboxaldehyd)

43 g 2,5,6,6-Tetramethylcyclohex-2-en-1-carboxaldehyd-N-methylaldimin wurden in 50 ml Wasser suspendiert und mit 50 %iger Schwefelsäure auf einen pH-Wert von 2,5 eingestellt. Das Reaktionsgemisch wurde 30 Minuten bei 30 bis 45 °C gerührt. Anschließend wurde es bei 30 bis 45 °C mit 50 %iger Natronlauge auf einen pH-Wert von 7,0 eingestellt. Das Gemisch wurde mit 100 ml n-Hexan extrahiert, die organische Phase mit wasserfreiem Natriumsulfat getrocknet und das Lösungsmittel bei 40 °C und 20 mbar abdestilliert.

Nach Destillation des so gewonnenen Rohproduktes resultierten 31,6 g 5-Methylcyclocitral als cis-trans-Isomerengemisch, entsprechend einer Ausbeute von 77 %. Nach gaschromatographischer Analyse lagen 71 % des α- (Kp. 40 bis 43 °C/0,3 mbar) und 16 % des β-Isomeren vor.

Beispiel 6

Herstellung von überwiegend 5-Methyl-β-cyclocitral (2,5,6,6-Tetramethylcyclohex-1-en-1-carboxaldehyd)

16,6 g 5-Methyl-α-cyclocitral wurden bei 0 bis 5 °C zu einer Lösung von 6,4 g KOH in 110 ml Methanol getropft, wonach das Reaktionsgemisch 80 Min. bei 0 bis 5 °C gehalten wurde. Anschießend wurde es auf 250 ml Eiswasser gegossen, mit Kochsalz gesättigt und 5 mal mit je 50 ml Ether extrahiert.

Die vereinigten Extrakte wurden 2 mal mit je 50 ml gesättigter Kochsalzlösung gewaschen und mit wasserfreiem Natriumsulfat getrocknet.

Das Lösungsmittel wurde bei 40 °C und 20 mbar abdestilliert. Es resultierten 14 g Rohprodukt, die nach H-NMR-Spektrum ca. 5 % des α- und 95 % des β-Isomeren (Kp. 64 bis 70 °C/0,3 mbar) enthielten, entsprechend einer Ausbeute von 84 %.

Beispiel 7

Herstellung eines Gemisches aus α-Cyclocitral-N-methylaldimin und β-Cyclocitral-N-methylaldimin durch Isomerisierung von α-Cyclocitral-N-methylaldimin

Zu einer Lösung von 10 g α-Cyclocitral-N-methylaldimin in 100 ml wasserfreiem Methanol wurden bei 0 °C 17 ml einer 30 %igen methanolischen Natriummethylatlösung getropft. Es wurde 2 Stunden bei 0 °C nachgerührt, 100 ml n-Hexan zugesetzt und die Mischung auf 500 g Eiswasser gegossen.

Die organische Phase wurde abgetrennt und die wäßrige Phase 3 mal mit je 50 ml n-Hexan extrahiert. Die vereinigten Extrakte wurden 2 mal mit je 250 ml n-Hexan gewaschen und getrocknet.

Nach Entfernung des Lösungsmittels blieben 7,5 g eines Öls zurück, das nach H-NMR-Spektrum zu ca. 75 % aus α-Cyclocitral-N-methyl-aldimin, zu 15 % aus β-Cyclocitral-N-methylaldimin und zu je 5 % aus α- und β-Cyclocitral bestand.

**Ansprüche**

1. Verfahren zur Herstellung von Cyclocitralen der allgemeinen Formel I

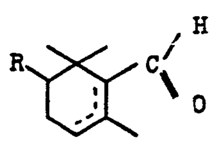

(I)

in welcher R für Wasserstoff oder die Methylgruppe steht und in welcher die gestrichelt gezeichneten Bindungen eine Doppelbindung in 1- oder 2-Stellung des Cyclohexenringes bedeuten, dadurch gekennzeichnet, daß man die offenkettigen Aldehyde der allgemeinen Formel III

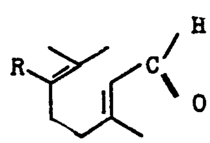

(III)

bei – 20 bis + 60 °C mit Methylamin zu deren N-Methylaldiminen umsetzt, diese bei – 30 bis 30 °C mit einem mehrfach molaren Überschuß von konzentrierter Schwefelsäure zu den Cyclocitral-N-methylaldiminen der allgemeinen Formel II

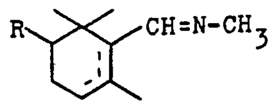

(II)

cyclisiert und die letztgenannten Verbindungen nach Abtrennung aus ihren Reaktionsgemischen oder ohne sie zuvor zu isolieren in an sich bekannter Weise zu den Cyclocitralen I hydrolysiert.

2. Cyclocitral-N-methyl-aldimine der allgemeinen Formel II

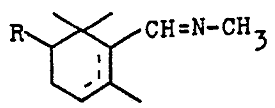

(II)

in der R für Wasserstoff oder die Methylgruppe steht und in welcher die gestrichelt gezeichneten Bindungen eine Doppelbindung in 1- oder 2-Stellung des Cyclohexenringes bedeuten.

**Claims**

1. A process for the preparation of a cyclocitral of the general formula I

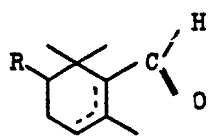

(I)

where R is hydrogen or methyl and where the bonds shown in broken lines indicate the presence of a double bond in the 1- or 2-position of the cyclohexene ring, wherein an open-chain aldehyde of the general formula III

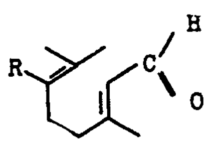

(III)

is reacted with methylamine at from – 20 to + 60 °C to give its N-methylaldimine, the latter is cyclized at from – 30 to 30 °C with a multi-molar excess of concentrated sulfuric acid to give the cyclocitral N-methylaldimine of the general formula II

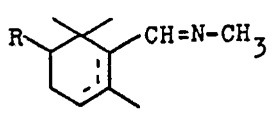

(II)

and the last-mentioned compound, with or without prior isolation from its reaction mixture, is hydrolyzed to the cyclocitral I in a conventional manner.

2. Cyclocitral N-methylaldimines of the general formula II

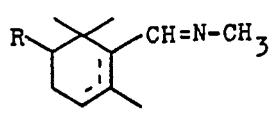

(II)

where R is hydrogen or methyl and where the bonds shown in broken lines indicate the presence of a double bond in the 1- or 2-position of the cyclohexene ring.

**Revendications**

1. Procédé pour la préparation de cyclocitrals de formule générale I

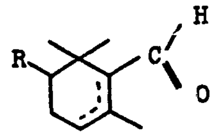

(I)

dans laquelle R est mis pour un hydrogène ou le groupe méthyle et dans laquelle les liaisons tracées en tirets représentent une double liaison en position 1 ou 2 du noyau cyclohexénique, caractérisé en ce qu'on fait réagir, à une température de − 20 à + 60 °C, les aldéhydes en chaîne ouverte de formule générale III

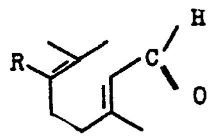

(III)

avec la méthylamine pour former leurs N-méthylaldimines, on cyclise celles-ci, à une température de − 30 à 30 °C, avec un excès plusieurs fois molaires d'acide sulfurique concentré, pour former les N-méthylaldimines de cyclocitral de formule générale II

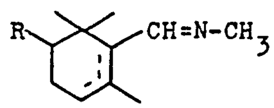

(II)

et on hydrolyse de façon connue en soi ces derniers composés, après séparation à partir de leurs mélanges réactionnels ou sans les isoler au préalable, pour obtenir les cyclocitrals I.

2. N-méthylaldimines de cyclocitral de formule générale II

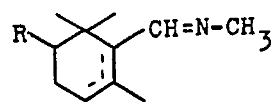

(II)

dans laquelle R est mis pour un hydrogène ou le groupe méthyle et dans laquelle les liaisons tracées en tirets représentent une double liaison en position 1 ou 2 du noyau cyclohexénique.